# EUROPÄISCHE PATENTANMELDUNG

(11) **EP 4 474 792 A2**
(43) Veröffentlichungstag der Anmeldung: **11.12.2024**
(21) Anmeldenummer: 24176144.4
(22) Anmeldetag: 15.05.2024
(51) Int. Cl.: G01N 1/22

(54) **VERFAHREN UND VORRICHTUNG ZUR ÜBERWACHUNG MIKROBIELLEN WACHSTUMS, VERWENDUNG EINES VERPACKTEN NÄHRMEDIUMS**

(30) Priorität: 17.05.2023 DE 102023113119
(71) Anmelder: SKAN AG, 4123 Allschwil (CH)
(72) Erfinder: Geiser, Christoph Joschi, 4103 Bottmingen (CH); Müller, Mathieu, 68510 Sierentz (FR); Tondera, Marc, 4125 Riehen (CH); Weiss, Florian Micha, 8064 Zürich (CH); Zeller, Mike, 4246 Wahlen (CH)
(74) Vertreter: Mertzlufft-Paufler, Cornelius

(57) **Zusammenfassung**

Erfindungsgemäß wird somit zur Überwachung mikrobiellen Wachstums vorgeschlagen, dass ein Fluidstrom über eine Fluidleitung aus einem Reinraum in eine kontrollierte Umgebung abgeführt wird, wobei in der kontrollierten Umgebung ein Nährmedium präsentiert und mit dem der Fluidstrom kontaktiert wird und anschließend so aus der kontrollierten Umgebung entnommen wird, dass ein Reinraumprozess nicht unterbrochen werden muss (Vgl. Fig. 10).

## Beschreibung

Die Erfindung betrifft ein Verfahren zur Überwachung auf mikrobiologische Verunreinigungen in einem Reinraum, vorzugsweise Isolator, wobei ein Fluidstrom, insbesondere eine Luftströmung, aus dem Reinraum abgeführt wird.

Die Erfindung betrifft weiter eine Vorrichtung zur Überwachung auf mikrobiologische Verunreinigungen in einem Reinraum.

Die Erfindung betrifft schließlich eine Verwendung, insbesondere innerhalb eines Reinraums und/oder innerhalb einer kontrollierten Umgebung, eines verpackten Nährmediums, insbesondere Impaktors, zur Präsentation des Nährmediums in einem Fluidstrom, wobei der Fluidstrom von einem Entnahmeort aus einem Reinraum, insbesondere Isolator, entnommen wurde, insbesondere wobei der Impaktor eine Düsenplatte hat, hinter der das Nährmedium angeordnet ist, wobei das Nährmedium, insbesondere hinter der Düsenplatte, vor Gebrauch durch einen Verschluss abgedeckt ist.

Es ist aus der Praxis bekannt, einen Reinraum auf mikrobiologische Verunreinigungen mittels Probenentnahme zu überwachen.

Der Erfindung liegt die Aufgabe zugrunde, eine Überwachung auf mikrobiologische Verunreinigungen innerhalb eines Reinraums nahezu kontinuierlich durchzuführen, so dass ein Reinraumprozess nicht oder nur kurzzeitig unterbrochen werden muss.

Zur Lösung dieser Aufgabe schlägt die Erfindung die Merkmale von Anspruch 1 vor. Insbesondere wird somit erfindungsgemäß bei einem Verfahren zur Überwachung auf mikrobiologische Verunreinigungen vorgeschlagen, dass der Fluidstrom ein Nährmedium in einer kontrollierten, außerhalb des Reinraums angeordneten, vorzugsweise in einer durch eine Fluidleitung verbundenen Umgebung und/oder von dem Reinraum abtrennbaren Umgebung kontaktiert, wobei das Nährmedium zur Überwachung auf mikrobiologische Verunreinigungen verwendet wird.

Dabei kann beispielsweise vorgesehen sein, dass die kontrollierte Umgebung, bis auf eine Zuführung, beispielsweise der Fluidleitung, von dem Reinraum abtrennbar ist. Besonders vorteilhaft ist dabei, dass somit ein Entnahmeort, von dem der Fluidstrom aus dem Reinraum, vorzugsweise Isolator, abgeführt werden kann, relativ frei festgelegt werden kann.

Besonders vorteilhaft ist zudem, dass verhindert werden kann, dass Erzeugnisse im Reinraum, insbesondere durch ein direktes Eingreifen in einen im Reinraum ablaufenden Reinraumprozess, kontaminiert werden. Somit kann ein praktisch unbegrenzter Austausch des Nährmediums ohne aktive Schleuse erfolgen, ohne dass der Reinraum anschließend dekontaminiert wird, beispielsweise dadurch, dass die kontrollierte Umgebung gegenüber dem Reinraum während des Austauschs abgeschlossen wird und die fluidische Verbindung nach dem Austausch für eine Messung hergestellt wird. Eine konstruktiv einfache Ausgestaltung kann eine Anordnung der kontrollierten Umgebung außerhalb des Reinraums sein. Dies kann eine Zugänglichkeit für den Austausch verbessern.

Ein Reinraumprozess kann dabei beispielsweise eine aseptische Abfüllung und/oder ein Verschließen und/oder eine Analyse von pharmazeutischen Erzeugnissen sein.

Das Nährmedium kann dabei beispielsweise fest, insbesondere als Agar, oder flüssig, insbesondere als Bouillon, sein.

Der Fluidstrom kann dabei beispielsweise horizontal und/oder vertikal oder schräg aus dem Reinraum abgeführt werden.

Allgemein kann der Reinraum bei einer vorteilhaften Anwendung der Erfindung beispielsweise als Isolator ausgebildet sein.

Besonders vorteilhaft ist dabei, dass über eine Kontaktierung des aus dem Reinraum abgeführten Fluidstroms mit dem Nährmedium in einer kontrollierten Umgebung verschiedene Dekontaminierungsprozesse minimiert werden.

Zudem ist besonders vorteilhaft, dass das Risiko einer Kontaminierung der Erzeugnisse, die im Reinraum während eines Reinraumprozesses hergestellt werden, ebenfalls minimiert werden kann.

Da die Überwachung auf mikrobiologische Verunreinigungen außerhalb des Reinraums stattfindet, ist besonders vorteilhaft, dass hierdurch eine nahezu kontinuierliche oder wiederkehrende Messung möglich ist, ohne dass der Reinraumprozess länger unterbrochen werden muss. Hierdurch können ebenfalls besonders vorteilhaft schnellere und kostengünstigere, mit geringerem Ausschuss zu realisierende Prozessabläufe generiert werden.

Weiterhin kann beispielsweise vorgesehen sein, dass wenigstens eine Kenngröße, insbesondere ein Volumenstrom des Fluidstroms, erfasst wird und/oder dass die Kenngröße, insbesondere aufgrund der Erfassung, reguliert wird.

Bei einer weiteren vorteilhaften Ausgestaltung kann vorgesehen sein, dass eine die kontrollierte Umgebung begrenzende Schnittstelle dekontaminiert wird, insbesondere bevor die Schnittstelle für den zu überwachenden Fluidstrom zugänglich gemacht wird.

Die kontrollierte Umgebung begrenzende Schnittstelle kann dabei beispielsweise das über eine Andocköffnung oder eine Zugangsöffnung ankoppelbare und/oder austauschbare Nährmedium sein.

Besonders vorteilhaft ist dabei, dass somit sichergestellt werden kann, dass möglicherweise nachweisbare mikrobiologische Verunreinigungen eindeutig dem aus dem Reinraum abgeführten Fluidstrom zugeordnet werden können und nicht durch eine Kontaminierung der Schnittstelle entstehen.

Somit kann vorteilhaft beispielsweise vermieden werden, dass vermehrungsfähige Organismen an der Schnittstelle, die aufgrund von Verunreinigungen außerhalb des Reinraums entstanden sind, als falsch positive Befunde zu einem unnötigen Ausschuss der während des Reinraumprozesses hergestellten Erzeugnisse resultieren.

Bei einer weiteren vorteilhaften Ausgestaltung kann vorgesehen sein, dass an der Fluidleitung eine Verzweigung oder eine Weiche ausgebildet ist, insbesondere wobei der Fluidstrom steuerbar ist.

Besonders vorteilhaft ist dabei, dass somit beispielsweise zwei separate Vorrichtungen zur Überwachung auf mikrobiologische Verunreinigungen verwendet werden können, die wiederum eine kontinuierliche Überwachung auf mikrobiologische Verunreinigungen im Reinraum ermöglichen. Diese können zeitlich überlappend oder zeitlich abwechselnd betrieben werden.

Dabei kann beispielsweise vorgesehen sein, dass ein Nährmedium in einer der beiden Vorrichtungen präsentiert und von dem aus dem Reinraum abgeführten Fluidstrom über die Fluidleitung kontaktiert wird. Sobald das Nährmedium beispielsweise in einen Inkubator gelegt und somit getauscht werden soll, kann ein Nährmedium in die andere Vorrichtung eingelegt und die Fluidleitung bzw. der Fluidstrom entsprechend umgelenkt werden. Somit kann vorteilhaft die Entnahme des einen Nährmediums simultan zur fortlaufenden Überwachung auf mikrobiologische Verunreinigungen des Fluidstroms aus dem Reinraum über das andere Nährmedium stattfinden.

Bei einer weiteren vorteilhaften Ausgestaltung kann vorgesehen sein, dass zur Dekontamination der Schnittstelle und/oder der kontrollierten Umgebung ein Dekontaminationsmittel, insbesondere ein Gas bzw. Aerosol, vorzugsweise Wasserstoffperoxid, zugeführt wird, insbesondere wobei das Gas bzw. Aerosol aus dem Reinraum und/oder aus einer separaten Dekontaminationsquelle zugeführt wird.

Besonders vorteilhaft ist dabei, dass das zur Dekontamination verwendete Gas bzw. Aerosol somit an unterschiedlichen Stellen der Schnittstelle zugeführt werden kann und die für die Begasung üblichen Loop-Verfahren anwendbar sind.

Beispielsweise kann dabei vorgesehen sein, dass das Gas bzw Aerosol in den Reinraum strömt, wobei die Dekontamination der Schnittstelle beispielsweise über den dem Reinraum abgeführten Fluidstrom erfolgt.

Bei einer weiteren vorteilhaften Ausgestaltung kann vorgesehen sein, dass der Fluidstrom an einer Prozessstelle innerhalb des Reinraums, an der vorzugsweise ein Reinraumprozess durchgeführt wird, aus dem Reinraum abgeführt wird, und/oder wobei der Fluidstrom in einer Fluidleitung, welche zumindest teilweise innerhalb des Reinraums verläuft, dem Nährmedium zugeführt wird. Besonders vorteilhaft ist dabei, dass über eine direkte Leitung des Fluidstroms eine kontinuierliche Messung, beispielsweise der Reinraumluft, bereits während des Reinraumprozesses möglich ist, wodurch der Reinraumprozess nicht oder nur kurzzeitig unterbrochen werden muss.

Bei einer weiteren vorteilhaften Ausgestaltung kann vorgesehen sein, dass der Fluidstrom durch einen Partikelzähler zur Erfassung einer Partikelanzahl und/oder Partikelgröße geführt wird, insbesondere wobei anschließend derselbe Fluidstrom zum Nährmedium geführt wird und/oder wobei ein Teilvolumen des Fluidstroms abgezweigt wird, insbesondere wobei der abgezweigte Fluidstrom an einen Partikelzähler zur Erfassung einer Partikelanzahl überführt wird und/oder wobei die Abzweigung außerhalb des Reinraums stattfindet.

Die Abzweigung des Fluidstroms kann dabei beispielsweise kontrolliert über regulierbare und/oder steuerbare Düsen erfolgen.

Dabei kann beispielsweise vorgesehen sein, dass der Partikelzähler kurz vor der kontrollierten Umgebung an einer Fluidleitung angeordnet ist. Weiterhin kann beispielsweise vorgesehen sein, dass der Partikelzähler außerhalb der kontrollierten Umgebung innerhalb des Reinraums an einer Fluidleitung angeordnet ist.

Besonders vorteilhaft ist dabei, dass die Überwachung auf Verunreinigungen durch funktionell unterschiedlichen Methoden um quantitative Angaben, beispielsweise Anzahl und/oder Größe der Verunreinigungen, ergänzt werden kann. Ein Partikelzähler kann auch zur genaueren Eingrenzung eines Zeitpunktes der Verunreinigung herangezogen werden.

Vorteilhaft ist zudem, dass über den Partikelzähler die Anzahl möglicher Verunreinigungen bestimmt werden kann, während über das Nährmedium detektiert werden kann, welche Art der Verunreinigung vorliegt.

Vorteilhaft ist somit, dass eine solche Kombination eine schnellere Reaktionszeit zur Unterbrechung des Reinraumprozesses ermöglicht.

Bei einer weiteren vorteilhaften Ausgestaltung kann vorgesehen sein, dass das Nährmedium von außen in die kontrollierte Umgebung eingebracht und an einer Aufnahme positioniert wird, insbesondere wobei nach dem Einbringen die kontrollierte Umgebung dekontaminiert wird.

Somit ist es beispielsweise möglich, das Nährmedium während eines Reinraumprozesses auszutauschen.

Besonders vorteilhaft ist dabei, dass das Nährmedium somit während eines Reinraumprozesses gewechselt bzw. ausgetauscht werden kann und der Reimraumprozess demnach unabhängig von dem Einbringen des Nährmediums ohne Risiko einer Kontaminierung des Reinraums fortgeführt werden kann. Demnach ist ebenfalls vorteilhaft, dass der Reinraumprozess bei einer Kontaminierung der kontrollierten Umgebung fortgeführt werden kann.

Bei einer vorteilhaften Ausgestaltung kann vorgesehen sein, dass vor einem Austausch des Nährmediums eine Rückströmverhinderung, insbesondere ein Ventil, vorzugsweise zwischen der kontrollierten Umgebung und dem Reinraum aktiviert wird.

Dabei kann beispielsweise vorgesehen sein, ein Ventil zu verwenden, welches minimale Kontaminierungsfläche bietet, um die Wahrscheinlichkeit einer Kontaminierung schlecht dekontaminierbarer Oberflächen zu verhindern.

Besonders vorteilhaft ist dabei, dass eine während des Austauschs des Nährmediums mögliche Kontaminierung der kontrollierten Umgebung zu keiner Kontaminierung der Fluidleitung durch eine Rückströmung des kontaminierten Fluidstroms führt.

Bei einer weiteren vorteilhaften Ausgestaltung kann vorgesehen sein, dass nach dem Einbringen ein Verschluss des Nährmediums entfernt und in der kontrollierten Umgebung aufbewahrt wird, insbesondere wobei der Verschluss außerhalb eines Strömungswegs des Fluidstroms aufbewahrt wird.

Hierbei kann beispielsweise vorgesehen sein, dass das Nährmedium beispielsweise an eine Austrittöffnung, einen Austrittraum oder einen Austrittskegel des Fluidstroms gepresst wird.

Besonders vorteilhaft ist dabei, dass der abgenommene Verschluss die Strömungseigenschaften des Fluidstroms somit nicht negativ beeinflusst, die beispielsweise in einer möglicherweise entstehenden turbulenten Strömung des Fluidstroms resultieren. Zudem ist hierdurch vorteilhaft verhindert, dass mögliche an dem Verschluss anhaftende Verunreinigungen von dem Fluidstrom mitgerissen werden und das Messergebnis verfälschen oder durch den Fluidstrom herangeführte Mikroorganismen am Verschluss oder einer Verschlussaufnahme anhaften und somit nicht dem Nährmedium überführt werden.

Die Verschlussaufnahme ist dabei insbesondere dazu ausgebildet, eine Außenseite eines Verschlusses zu umgreifen. Wird die Außenseite des Verschlusses hierbei vollständig oder praktisch vollständig von der Verschlussaufnahme abgedeckt, so kann eine Kontamination durch die Außenseite ausgeschlossen (bzw. praktisch ausgeschlossen) werden. Beispielsweise kann dies nach Art eines Rapid Transfer Ports realisiert sein.

Alternativ oder zusätzlich sind zur Lösung der genannten Aufgabe erfindungsgemäß die Merkmale des nebengeordneten, auf ein Verfahren gerichteten Anspruchs vorgesehen. Insbesondere wird somit zur Lösung der genannten Aufgabe bei einem Verfahren zur Überwachung auf mikrobiologische Verunreinigungen in einem Reinraum, wobei ein Fluidstrom, insbesondere eine Luftströmung, aus dem Reinraum abgeführt wird, wobei der Fluidstrom ein Nährmedium kontaktiert, wobei das Nährmedium zur Überwachung auf mikrobiologische Verunreinigungen verwendet wird, insbesondere wie zuvor beschrieben, vorgeschlagen, dass eine Fluidleitung, die den Fluidstrom zum Nährmedium bringt, an das Nährmedium angeschlossen wird, nachdem ein Verschluss des Nährmediums entfernt wurde.

Dabei kann beispielsweise vorgesehen sein, dass das Anschließen der Fluidleitung an das Nährmedium ein mechanisches Verbinden und/oder ein Öffnen einer Absperrung ist.

Besonders vorteilhaft ist dabei, dass ein kontrollierter Raum von dem Reinraum gebildet werden kann und somit auf einen von dem Reinraum separat angeordneten kontrollierten Raum verzichtet werden kann.

Vorteilhaft ist zudem, dass hierdurch ein Rückströmen eines möglicherweise kontaminierten Fluidstroms verhindert wird, da die Fluidleitung erst an das Nährmedium angeschlossen wird, wenn das Nährmedium verschlossen ist.

Bei einer weiteren vorteilhaften Ausgestaltung kann vorgesehen sein, dass der Verschluss über eine Transferbewegung, insbesondere eine vorzugsweise horizontale Schwenkbewegung und/oder eine Hubbewegung, entfernt wird.

Besonders vorteilhaft ist dabei, dass der Verschluss somit problemlos in einen Bereich außerhalb des Strömungswegs des Fluidstroms bewegt und gleichzeitig von dem Nährmedium entfernt werden kann.

Bei einer weiteren vorteilhaften Ausgestaltung kann vorgesehen sein, dass das Nährmedium, insbesondere auf einem Nährmediumsträger, zur Entfernung und/oder zur Anbringung des Verschlusses und/oder zur Ankopplung der Fluidleitung von einer Hebevorrichtung, insbesondere die mit einer Membrane verbindbar oder verbunden ist, vorzugsweise vertikal bewegt wird.

Der Nährmediumsträger kann dabei beispielsweise ein Gehäuse für das Nährmedium bilden.

Dabei kann beispielsweise vorgesehen sein, dass die Membrane einen Raum, in den der Verschluss beispielsweise verschwenkt wird, von einem Außenraum zu trennen.

Besonders vorteilhaft ist dabei, dass an diesem Außenraum eine Dekontaminierung erfolgen kann.

Bei einer weiteren vorteilhaften Ausgestaltung kann vorgesehen sein, dass das Nährmedium nach erfolgter Kontaktierung mit dem Fluidstrom aus der kontrollierten Umgebung entfernt wird, insbesondere durch eine Bewegung der Aufnahme.

Dabei kann beispielsweise vorgesehen sein, dass die Aufnahme horizontal und/oder vertikal bewegt wird.

Beispielsweise kann zudem vorgesehen sein, dass das Nährmedium nach erfolgter Kontaktierung mit dem Fluidstrom mit dem Verschluss verschlossen wird, bevor das Nährmedium aus der kontrollierten Umgebung entfernt wird.

Besonders vorteilhaft ist dabei, dass hierdurch eine Kontaminierung des kontrollierten Raums weitestgehend verhindert werden kann.

Vorteilhaft ist somit, dass das Nährmedium somit problemlos entfernt und beispielsweise einem Inkubator zugeführt werden kann.

Bei einer weiteren vorteilhaften Ausgestaltung kann vorgesehen sein, dass in dem Fluidstrom oder einem weiteren Fluidstrom eine Partikeldetektion durchgeführt wird und ein oder das Nährmedium erst und/oder automatisch in dem Fluidstrom präsentiert wird, wenn die Partikeldetektion ein vorzugsweise vermehrungsfähiges und/oder lebendes Objekt detektiert hat, insbesondere durch Umleiten des Fluidstroms, Öffnen einer den Fluidstrom leitenden Fluidleitung und/oder Starten des Fluidstroms.

Besonders vorteilhaft ist dabei, dass das Nährmedium somit insbesondere erst dann geöffnet, verwendet und/oder verbraucht wird, wenn von dem Partikelzähler nachweislich mikrobiologische Verunreinigungen detektiert werden konnten.

Somit kann beispielsweise ebenfalls vorteilhaft ein unnötiges Austrocknen des Nährmediums verhindert werden.

Alternativ oder zusätzlich sind zur Lösung der genannten Aufgabe erfindungsgemäß die Merkmale des nebengeordneten, auf eine Vorrichtung gerichteten Anspruchs vorgesehen. Insbesondere wird somit erfindungsgemäß zur Lösung der genannten Aufgabe zur Überwachung auf mikrobiologische Verunreinigungen in einem Reinraum vorgeschlagen, dass eine Einhausung, die eine, beispielsweise die bereits erwähnte, kontrollierte Umgebung umschließt, eine, beispielsweise die bereits erwähnte, Aufnahme zum Präsentieren eines, beispielsweise des bereits erwähnten, Nährmediums in der kontrollierten Umgebung, eine fluidische Anbindung zur Verbindung mit dem Reinraum und eine Schnittstelle, welche die kontrollierte Umgebung begrenzt, insbesondere zur Bereitstellung des Nährmediums, umfasst.

Besonders vorteilhaft ist dabei, dass somit die Vorteile des bereits beanspruchten Verfahrens realisiert werden können.

Bei einer vorteilhaften Ausgestaltung kann vorgesehen sein, dass die fluidische Anbindung eine Verzweigung oder eine Weiche hat, die dazu ausgebildet ist, einen Fluidstrom umzulenken.

Besonders vorteilhaft ist dabei, dass ein Fluidstrom, der insbesondere aus dem Reinraum abgeführt wird, kontinuierlich, beispielsweise durch die Verwendung von zwei separaten kontrollierten Umgebungen, in denen ein Nährmedium präsentiert werden kann, auf mikrobiologische Verunreinigungen überwacht werden kann.

Bei einer weiteren vorteilhaften Ausgestaltung kann vorgesehen sein, dass eine Dekontaminationsvorrichtung ausgebildet ist, durch welche zumindest die Schnittstelle, insbesondere die kontrollierte Umgebung, dekontaminierbar ist.

Dabei kann beispielsweise vorgesehen sein, dass die Dekontaminationsvorrichtung ausgebildet ist, zur Dekontaminierung der Schnittstelle ein Dekontaminationsmittel, insbesondere Gas bzw. Aerosol, beispielsweise Wasserstoffperoxid, beispielsweise aus dem Reinraum und/oder aus einer separaten Dekontaminationsquelle der Schnittstelle zuzuführen.

Besonders vorteilhaft ist dabei, dass es somit nahezu verhinderbar ist, dass mikrobiologische Verunreinigungen durch eine kontaminierte Schnittstelle entsteht. Hierdurch ist wiederum vorteilhaft, dass mögliche detektierte mikrobiologische Verunreinigungen mit hoher Wahrscheinlichkeit durch eine Kontaminierung des Reinraums entstanden sind.

Bei einer weiteren vorteilhaften Ausgestaltung kann vorgesehen sein, dass die Schnittstelle als Teil der Einhausung, insbesondere als Teil einer Verschlussvorrichtung der Einhausung ausgebildet ist.

Dabei kann beispielsweise vorgesehen sein, dass die Schnittstelle von einem Verschluss des Nährmediums gebildet wird.

Vorteilhaft ist dabei, dass somit die Möglichkeit besteht, das Nährmedium vor einer Entnahme zu verschließen.

Bei einer weiteren vorteilhaften Ausgestaltung kann vorgesehen sein, dass die kontrollierte Umgebung eine Zuleitung und/oder eine Ableitung für ein Dekontaminationsmittel der Dekontaminationsvorrichtung hat und/oder wobei die Dekontaminationsvorrichtung als Teil der Vorrichtung ausgebildet ist.

Besonders vorteilhaft ist dabei, dass somit unterschiedliche Methoden zur Dekontaminierung, beispielsweise der Schnittstelle, anwendbar sind.

Dabei kann beispielsweise vorgesehen sein, dass das Dekontaminationsmittel über einen Fluidstrom, welcher aus dem Reinraum abgeführt wird, der kontrollierten Umgebung zugeführt wird.

Bei einer weiteren vorteilhaften Ausgestaltung kann vorgesehen sein, dass die Aufnahme in der kontrollierten Umgebung ausgebildet ist, insbesondere wobei die Aufnahme dazu eingerichtet ist, die Vorrichtung nach außen zu öffnen.

Die Aufnahme kann dabei beispielsweise horizontal und/oder vertikal beweglich sein.

Zudem ist vorteilhaft, dass ein Wechsel des Nährmediums somit unabhängig eines im Reinraum ablaufenden Reinraumprozesses durchgeführt werden kann.

Bei einer weiteren vorteilhaften Ausgestaltung kann vorgesehen sein, dass die Vorrichtung eine Hebevorrichtung aufweist.

Dabei kann beispielsweise vorgesehen sein, dass die Aufnahme vertikal beweglich ist.

Besonders vorteilhaft ist dabei, dass somit beispielsweise eine einfache Möglichkeit zur Entnahme des Nährmediums realisiert ist. Bei einer Ausgestaltung, für die eigenständig Schutz beansprucht wird, kann erfindungsgemäß vorgesehen sein, dass zur Überwachung auf mikrobiologische Verunreinigungen in einem Reinraum, insbesondere zur Durchführung eines bereits genannten Verfahrens und/oder nach einem der auf eine Vorrichtung gerichteten Ansprüche, umfassend die oder eine Aufnahme zum Präsentieren eines oder des Nährmediums, eine fluidische Anbindung, insbesondere von einem Entnahmeort im Reinraum, die an das Nährmedium ankoppelbar ist und ein Mittel zum vorzugsweise ferngesteuerten Entfernen eines Verschlusses des Nährmediums bevor die fluidische Anbindung angekoppelt ist.

Von Vorteil ist dabei, dass eine Auswechslung des Nährmediums, das mit einem Verschlussgekapselt bereitgestellt werden kann, durchführbar ist, ohne dass ein Inneres des Reinraums gestört werden muss.

Das Entfernen kann vorzugsweise automatisiert oder manuell erfolgen. Dies kann beispielsweise vollautomatisch oder manuell über eine Fernsteuerung erfolgen.

Eine Ankopplung kann dabei beispielsweise ein mechanisches Verbinden zwischen dem Nährmedium und der fluidischen Anbindung oder beispielsweise das Öffnen einer Absperrung sein.

Besonders vorteilhaft ist dabei, dass somit verhindert werden kann, dass ein kontaminierter Fluidstrom in den Reinraum strömt.

Vorteilhaft ist zudem, dass die kontrollierte Umgebung somit von dem Reinraum selbst gebildet werden kann.

Bei einer weiteren vorteilhaften Ausgestaltung kann vorgesehen sein, dass eine elastische Membrane außerhalb eines Strömungswegs des Fluidstroms zwischen dem Isolator und der kontrollierten Umgebung ausgebildet ist.

Der Strömungswegs ist dabei beispielsweise der Strömungsbereich, den ein Fluid während einer Fluidströmung durchläuft.

Besonders vorteilhaft ist, dass die Membrane mit dem Strömungsbereich, während ein Fluidstrom aus dem Reinraum in die kontrollierte Umgebung abgeführt wird, fluidisch verbunden ist. Weiterhin ist vorteilhaft, dass die Membrane aufgrund ihrer Elastizität zudem ermöglicht, dass, sobald kein Fluidstrom mehr abgeführt wird, die fluidische Verbindung gelöst werden kann.

Somit ist es beispielsweise möglich, dass, beispielsweise bei einer vertikalen Bewegung der Membrane, den Verschluss des Nährmediums beispielsweise durch eine Schwenkbewegung entfernt werden kann.

Bei einer weiteren vorteilhaften Ausgestaltung kann vorgesehen sein, dass die Membrane als Teilabschnitt der oder einer Einhausung der oder einer kontrollierten Umgebung oder des Reinraums ausgebildet ist.

Somit kann ein beweglicher Abschluss der kontrollierten Umgebung oder des Reinraumes, insbesondere Isolators, erreichbar sein, insbesondere zu einer Auswechslung des Nährmediums.

Bei einer weiteren vorteilhaften Ausgestaltung kann vorgesehen sein, dass die Vorrichtung eine Transfervorrichtung, insbesondere Schwenkvorrichtung, aufweist, die mit einer Verschlussaufnahme ausgebildet ist.

Dabei kann beispielsweise vorgesehen sein, dass der Verschluss des Nährmediums entfernt werden kann.

Der Verschluss kann dabei beispielsweise ein aufgesetzter Deckel oder eine Folie sein.

Besonders vorteilhaft ist dabei, dass der Verschluss somit außerhalb des Strömungswegs des Fluidstroms platziert werden kann.

Beispielsweise kann vorgesehen sein, dass die Verschlussaufnahme beispielsweise den Verschluss des Nährmediums umgreift.

Bei einer weiteren vorteilhaften Ausgestaltung kann vorgesehen sein, dass die Vorrichtung die oder eine Fluidleitung umfasst, wobei insbesondere die Schnittstelle als Teil der Fluidleitung ausgebildet ist, und/oder wobei ein Strömungsweg des Fluidstroms durch die Fluidleitung zwischen einer der Vorrichtung abgewandten Öffnung der Fluidleitung und der Aufnahme weniger als ein Meter, vorzugsweise weniger als zwei oder drei Meter, insbesondere weniger als vier Meter beträgt.

Besonders vorteilhaft ist dabei, dass über einen möglichst kurzen Strömungsweg der Fluidstrom möglichst schnell von dem Reinraum in die kontrollierte Umgebung abgeführt werden kann.

Bei einer weiteren vorteilhaften Ausgestaltung kann vorgesehen sein, dass die Fluidleitung schließbar ist, insbesondere, wenn die Transfervorrichtung in einer entfernten Position angeordnet ist.

Besonders vorteilhaft ist dabei, dass sich Verunreinigungen nicht von dem kontrollierten Volumen in den Reinraum ausbreiten können.

Bei einer weiteren vorteilhaften Ausgestaltung kann vorgesehen sein, dass im Strömungsweg vorgeschaltet dem Nährmedium der oder ein Partikelzähler angeordnet ist und/oder dass der Fluidstrom abgezweigt wird.

Dabei ist beispielsweise vorgesehen, dass ein Strömungsteiler ausgebildet ist.

Dabei kann beispielsweise vorgesehen sein, dass der Partikelzähler kurz vor der kontrollierten Umgebung ausgebildet ist. Zudem kann beispielsweise ebenfalls vorgesehen sein, dass der Partikelzähler innerhalb des Reinraums ausgebildet ist.

Besonders vorteilhaft ist dabei, dass somit eine Methode zur Überwachung auf mikrobiologische Verunreinigungen durch quantitative Analysen ergänzbar ist.

Vorteilhaft ist zudem, dass der Partikelzähler Auskunft darüber geben kann, wie viele Verunreinigungen vorhanden sind, während das Nährmedium detektieren kann, welche Art von Verunreinigungen detektiert wurden.

Bei einer weiteren vorteilhaften Ausgestaltung kann vorgesehen sein, dass die fluidische Anbindung mit einer Einrichtung zur Rückströmverhinderung, insbesondere einem Ventil, ausgebildet ist.

Beispielsweise kann ein Ventil verwendet werden, welches möglichst wenig Kontaminierungsfläche bzw. möglichst wenig schlecht dekontaminierbare Flächen hat.

Somit kann vorteilhaft vermieden werden, dass ein kontaminierter Fluidstrom in den Reinraum strömt, wodurch verhindert wird, dass der Reinraum dekontaminiert und der Reinraumprozess unterbrochen werden muss.

Bei einer weiteren vorteilhaften Ausgestaltung kann vorgesehen sein, dass die Vorrichtung eine Pumpe, insbesondere zum Einbringen des Fluidstroms in die kontrollierte Umgebung aufweist.

Besonders vorteilhaft ist dabei, dass somit die Strömungsrichtung des Fluidstroms beeinflusst werden kann. Somit ist es beispielsweise möglich, den Fluidstrom horizontal und/oder vertikal aus dem Reinraum abzuführen.

Bei einer weiteren vorteilhaften Ausgestaltung kann vorgesehen sein, dass die Vorrichtung wenigstens einen Sensor zur Erfassung der oder einer Kenngröße des Fluidstroms hat, und/oder wobei die Vorrichtung eine Steuervorrichtung zur Regulierung des Fluidstroms hat.

Besonders vorteilhaft ist dabei, dass somit die Strömungseigenschaften des Fluidstroms beeinflussbar und/oder entsprechend der jeweiligen Bedingungen anpassbar sind.

Besonders vorteilhaft ist zudem, dass somit verhindert werden kann, dass die Strömungsgeschwindigkeit des Fluids zu hoch oder zu gering ist, wodurch wiederum keine genaue Detektierung von dem den Fluidstrom enthaltenen Verunreinigungen über das Nährmedium möglich ist.

Alternativ oder zusätzlich sind zur Lösung der genannten Aufgabe erfindungsgemäß die Merkmale des nebengeordneten, auf eine Verwendung eines verpackten Nährmediums gerichteten Anspruchs vorgesehen. Insbesondere wird somit zur Lösung der genannten Aufgabe bei einer Verwendung, insbesondere innerhalb eines Reinraums und/oder innerhalb einer kontrollierten Umgebung, eines verpackten Nährmediums, insbesondere Impaktors, zur Präsentation des Nährmediums in einem Fluidstrom, wobei der Fluidstrom von einem Entnahmeort aus einem Reinraum, insbesondere Isolator, entnommen wurde, insbesondere wobei der Impaktor eine Düsenplatte hat, hinter der das Nährmedium angeordnet ist, wobei das Nährmedium, insbesondere hinter der Düsenplatte, vor Gebrauch durch einen Verschluss, insbesondere einen Deckel, abgedeckt ist, erfindungsgemäß vorgeschlagen, dass der Verschluss mit einer Verschlussaufnahme gegriffen und von dem Nährmedium, insbesondere der Düsenplatte, entfernt wird und dass eine fluidische Anbindung von dem Entnahmeort zu dem Nährmedium, insbesondere der Düsenplatte, hergestellt wird.

Beispielsweise kann die Verwendung innerhalb eines Reinraums, insbesondere Impaktors, und/oder innerhalb einer kontrollierten Umgebung stattfinden.

Weiterhin kann beispielsweise vorgesehen sein, dass das Nährmedium, insbesondere die Düsenplatte, nach Gebrauch in umgekehrter Richtung von dem Verschluss, welcher beispielsweise von der Verschlussaufnahme gegriffen wird, verschlossen wird.

Der Impaktor ist vorzugsweise dazu ausgebildet, den Fluidstrom, insbesondere über die Düsenplatte, zu beschleunigen. Die Düsenplatte ist dabei vorzugsweise mit in Umfangsrichtung angeordneten Schlitzen ausgebildet, die ein so geringes Öffnungsmaß haben, dass Verunreinigungen nur durch einen Unterdruck hindurchdringen können. Besonders vorteilhaft ist dabei, dass somit eine Kontaktierung des aus dem Reinraum abgeführten Fluidstroms mit dem Nährmedium möglich ist, wodurch wiederum eine Überwachung auf mikrobiologische Verunreinigungen realisiert werden kann.

Bei einer weiteren vorteilhaften Ausgestaltung kann vorgesehen sein, dass der Reinraum und/oder die kontrollierte Umgebung eine mit der Verschlussaufnahme, welche eine Außenseite des Verschlusses abdeckt, verschließbare Öffnung hat.

Besonders vorteilhaft ist dabei, dass das Nährmedium zur Entnahme mit einem in dem Reinraum bzw. der kontrollierten Umgebung befindlichen Verschluss verschlossen werden kann. Somit kann verhindert werden, dass Verunreinigungen nach der Entnahme in das Nährmedium gelangen. Die Verwendung einer Verschlussaufnahme, mit der eine Öffnung verschließbar ist, ermöglicht hier, dass das Verschließen und die Entnahme des mit dem Verschluss verschlossenen Nährmediums durch die Öffnung möglich sind, ohne dass der Reinraum bzw. die kontrollierte Umgebung gestört werden.

Weiterhin ist vorteilhaft, dass somit sichergestellt werden kann, dass lediglich der Fluidstrom das Nährmedium kontaktiert, der aus dem Reinraum abgeführt wird. Vorteilhaft kann demnach verhindert werden, dass ein Fluidstrom, der nicht aus dem Reinraum abgeführt wird, das Nährmedium kontaktiert und zu einem verfälschten Messergebnis führt.

Bei einer weiteren vorteilhaften Ausgestaltung kann vorgesehen sein, dass der Impaktor nach einem Exponieren des Nährmediums von dem Fluidstrom entfernt und einer Inkubation zugeführt wird, insbesondere wobei vor dem Entfernen das Nährmedium verschlossen wird.

Besonders vorteilhaft ist dabei, dass somit mikrobiologische Verunreinigungen nachgewiesen werden können. Vorteilhaft ist zudem, dass bei einer Verunreinigung über das Verschließen des Nährmediums vor der Entfernung verhindert werden kann, dass weitere Flächen kontaminiert werden.

Bei einer weiteren vorteilhaften Ausgestaltung kann vorgesehen sein, dass die Verschlussaufnahme vor einer Kontaktierung des Verschlusses einen Abschluss einer kontrollierten Umgebung bildet, insbesondere auf einer von dem Verschluss abgewandten Seite.

Besonders vorteilhaft ist dabei, dass die Verschlussaufnahme somit eine zusätzliche Abgrenzung zu dem Reinraum bildet und die Wahrscheinlichkeit einer Kontaminierung des Reinraums wiederum verringert werden kann. Zudem ist vorteilhaft, dass Verunreinigungen des Verschlusses nicht unbedingt in einer Verunreinigung des kontrollierten Raums resultieren.

Bei einer weiteren vorteilhaften Ausgestaltung kann vorgesehen sein, dass die fluidische Anbindung ein Leitungsstück umfasst.

Besonders vorteilhaft ist dabei, dass der Fluidstrom somit aus dem Reinraum in die kontrollierte Umgebung abgeführt werden kann.

Bei einer weiteren vorteilhaften Ausgestaltung kann vorgesehen sein, dass die fluidische Anbindung mittels einer Kupplung hergestellt wird, die eine Relativbewegung zwischen der Düsenplatte und einem Leitungsstück ausführt.

Die Kupplung kann dabei beispielsweise die fluidische Anbindung zwischen einer Unterseite des Leitungsstücks und einer Oberseite der Düsenplatte sein.

Besonders vorteilhaft ist dabei, dass somit eine lösbare Verbindung zwischen der Düsenplatte und dem Leitungsstück möglich ist, so dass vorteilhaft die Leitungsstücke ausgetauscht und/oder gewechselt werden können.

Besonders vorteilhaft ist zudem, dass somit sichergestellt werden kann, dass über die Kupplung lediglich der Fluidstrom das Nährmedium kontaktiert, der aus dem Reinraum abgeführt wird.

Die Erfindung wird nun anhand von Ausführungsbeispielen näher beschrieben, ist jedoch nicht auf die Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen des Ausführungsbeispiels.

Es zeigt in jeweils stark vereinfachter Form
- Fig. 1: eine dreidimensionale Schnittdarstellung einer Vorrichtung zur Überwachung auf mikrobiologische Verunreinigungen mit einem Nährmediumsträger, in dem sich das Nährmedium befindet, einer kontrollierten Umgebung, einer Aufnahme, einem Verschluss, und einem Strömungsweg mit einer Fluidleitung,
- Fig. 2: eine dreidimensionale Darstellung einer weiteren erfindungsgemäßen Vorrichtung mit einer horizontal und vertikal beweglichen Verschlussaufnahme und einer horizontal beweglichen Fluidleitblende,
- Fig. 3: eine dreidimensionale Darstellung einer alternativen Ausführung einer Vorrichtung nach Fig. 2,
- Fig. 4: eine dreidimensionale Darstellung einer Vorrichtung nach Fig. 3, wobei die Aufnahme und die Fluidleitblende vertikal beweglich ist,
- Fig. 5: eine zweidimensionale Schnittdarstellung in einer Frontansicht nach Fig. 4,
- Fig. 6: eine dreidimensionale Darstellung einer Vorrichtung mit elastischer Membrane,
- Fig. 7: eine zweidimensionale Schnittdarstellung nach Fig. 6, wobei die Aufnahme nicht anliegt und auf dem Nährmedium ein Verschluss ausgebildet ist,
- Fig. 8: eine zweidimensionale Schnittdarstellung nach Fig. 6, wobei das Nährmedium an einer Verschlussaufnahme anliegt zur Entfernung des Verschlusses des Nährmediums,
- Fig. 9: eine zweidimensionale Schnittdarstellung nach Fig. 6, wobei der Verschluss von dem Nährmedium entfernt ist und die elastische Membrane mit einem Nährmediumsträger abgesenkt ist,
- Fig. 10: eine zweidimensionale Schnittdarstellung nach Fig. 6, wobei der Verschluss innerhalb einer kontrollierten Umgebung außerhalb eines Strömungswegs eines Fluidstroms positioniert ist und der Nährmediumsträger an die Fluidleitung angeschlossen ist,
- Fig. 11: eine dreidimensionale Darstellung der Vorrichtung nach Fig. 6, zusätzlich mit einer Bodenplatte des Reinraums und vor der Bodenplatte des Reinraums an einer Fluidleitung positioniertem Partikelzähler,
- Fig. 12: eine dreidimensionale Darstellung analog Fig. 11, wobei der Partikelzähler innerhalb des Reinraums oberhalb der Bodenplatte des Reinraums an der Fluidleitung positioniert ist,
- Fig. 13: eine schematische Darstellung eines Aufbaus zur kontinuierlichen Überwachung auf mikrobiologische Verunreinigungen mit einer Weiche und zwei separaten Vorrichtungen,
- Fig. 14: eine schematische Darstellung nach Fig. 13 ohne Weiche und mit eingebauten Ventilen zur Fluidstromführung und Verzweigung und
- Fig. 15: die modifizierte Vorrichtung gemäß Fig. 6 im eingebauten Zustand in einem Reinraum,
- Fig. 16: eine zweidimensionale Darstellung einer weiteren erfindungsgemäßen Vorrichtung zur Überwachung auf mikrobiologische Verunreinigungen mit einer seitlich klappbaren Schnittstelle.
- Fig. 17: eine zweidimensionale Darstellung einer weiteren erfindungsgemäßen Vorrichtung zur Überwachung auf mikrobiologische Verunreinigungen mit paralleler Anordnung des Partikelzählers und des Nährmediums.

Eine im Ganzen mit 1 bezeichnete Vorrichtung zur Überwachung auf mikrobiologische Verunreinigungen hat eine Verschlussaufnahme 2 für einen Verschluss 3, welche auf einem Nährmediumsträger 11 platzierbar ist, wobei der Nährmediumsträger 11 ein Nährmedium 4 präsentiert und einen Impaktor 29, der von einer Einhausung 13 teilweise umschlossen ist. Die Einhausung 13 hat eine mit der Verschlussaufnahme 2, welche eine Außenseite des Verschlusses 3 abdeckt, verschließbare Öffnung 24.

Das Nährmedium 4 ist dabei hinter einer Düsenplatte 25 angeordnet. Der Impaktor 29 ist über einen Sauganschluss 30 mit einer nicht weiter dargestellten Pumpe 35 verbunden. Die Pumpe saugt somit Fluid aus einer Fluidleitung 7 durch die Düsenplatte 25 an dem Nährmedium 4 vorbei. Mitgetragene Verunreinigungen lagern sich hierbei auf dem Nährmedium 4 ab und können anschließend nachgewiesen werden.

Über der auf dem Nährmedium 4 aufgesetzten Verschlussaufnahme 2 ist ein sich radial erweiternder Strömungskanal mit einer Fluidleitung 7 und einer fluidischen Anbindung 27, die ein Leitungsstück 26 umfasst, ausgebildet. Die Fluidleitung 7 kann dabei an einer Prozessstelle innerhalb eines Reinraums 20, an der vorzugsweise ein Reinraumprozess durchgeführt wird und/oder teilweise innerhalb des Reinraums 20 verlaufen. Hier ist vorgesehen, dass der Reinraum 20 oberhalb der Fluidleitung 7 angeordnet ist.

Somit kann ein Fluidstrom 17 aus dem Reinraum 20 von einem Entnahmeort 28, beispielsweise über die bereits erwähnte Pumpe 35, vertikal über die Fluidleitung 7 und einen Strömungskanal, der einen Strömungsweg der Fluidströmung 17 definiert, abgeführt und einer kontrollierten Umgebung 5 zugeführt werden. Der Fluidstrom 17 wird dabei von der Düsenplatte 25, die mit in Umfangsrichtung angeordneten Schlitzen mit lichtem Öffnungsmaß ausgebildet ist, beschleunigt.

Eine kontrollierte Umgebung 5 ist von einer Schnittstelle 6 begrenzt, die zunächst von einem Gas oder Aerosol, insbesondere von einem Wasserstoffperoxid enthaltenden Gas, dekontaminiert wird, bevor die Schnittstelle 6 für den zu beprobenden oder zu überwachenden Fluidstrom 17 zugänglich gemacht wird und nachdem ein Nährmediumsträger 11, der für die Dekontamination kein Nährmedium 4 enthält, mit der kontrollierten Umgebung 5 verbunden wurde.

Das Gas oder Aerosol wird dabei aus dem Reinraum 20 und/oder aus einer separaten Dekontaminationsquelle, insbesondere einer Dekontaminationsvorrichtung, zugeführt. Nach der Dekontamination wird der Nährmediumsträger 11 gegen einen Nährmediumsträger 11 mit Nährmedium 4 getauscht.

In der kontrollierten Umgebung 5 befindet sich somit das Nährmedium 4, welches von einer Aufnahme 8 auf einem Nährmediumsträger 11 präsentiert wird.

Zur Überwachung auf mikrobiologische Verunreinigungen ist vorgesehen, dass zunächst der Verschluss 3 des Nähmediums 4 über eine Transferbewegung der Verschlussaufnahme 2, welche eine Außenseite des Verschlusses 3 umfasst, entfernt wird.

Es kann somit gesagt werden, dass die Fluidleitung 7 erst fluidisch an das Nährmedium 4 angeschlossen wird, nachdem der Verschluss 3 des Nährmediums 4 entfernt wurde.

Somit kann anschließend der Fluidstrom 17 das Nährmedium 4 außerhalb des Reinraums 20 in der dekontaminierten kontrollierten Umgebung 5 kontaktieren.

Zur Entnahme des Nährmediums 4 ist in diesem Ausführungsbeispiel vorgesehen, dass sich eine Aufnahme 8 der Vorrichtung 1 nach außen über eine Transferbewegung öffnen lässt.

Bei einem Austausch des Nährmediums 4 wird beispielsweise ein in dieser Figur nicht gezeigtes Ventil aktiviert, wodurch eine Rückströmung des Fluidstroms 17 verhinderbar ist.

Nach der Kontaktierung des aus dem Reinraum 20 abgeführten Fluidstroms 17 mit dem Nährmedium 4 kann somit auf mikrobiologische Verunreinigungen überwacht werden.

Dies ist dadurch realisierbar, dass der Impaktor 29 nach einem Exponieren des Nährmediums 4 von dem Fluidstrom 17 entfernt und nachdem der Verschluss 3 des Nährmediums 4 wieder über eine Transferbewegung der Verschlussaufnahme 2 auf das Nährmedium 4 aufgesetzt wurde, einer Inkubation zugeführt wird.

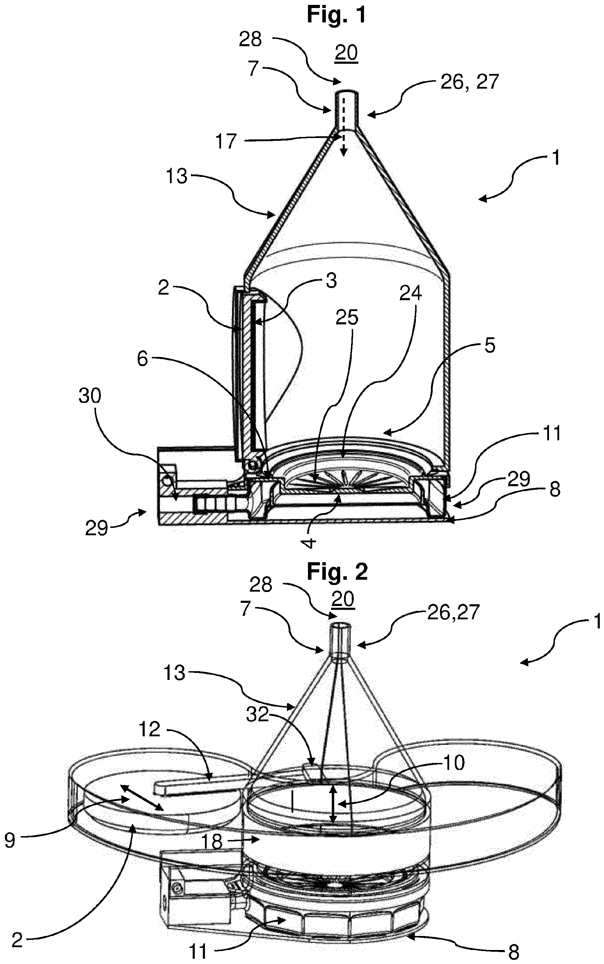

Fig. 2 zeigt im Unterschied zu Fig. 1 eine weitere Vorrichtung 1 zur Überwachung auf mikrobiologische Verunreinigungen aus dem Reinraum 20.

Die Verschlussaufnahme 2, die zur Aufnahme des Verschlusses 3 des Nährmediums 4 ausgebildet ist, wird hierbei über eine Hebevorrichtung 32 bzw. Schwenkvorrichtung 12, die der Verschlussaufnahme 2 ermöglicht, eine Hubbewegung 10 und/oder eine Schwenkbewegung 9 auszuführen, an das Nährmedium 4 angebracht, wodurch der Verschluss 3 des Nährmediumträgers 11 entfernt oder auf den Nährmediumsträger 11 aufgesetzt wird.

Zusätzlich ist in diesem Ausführungsbeispiel eine Fluidleitblende 18 gezeigt, die dazu ausgebildet ist, eine horizontale Schwenkbewegung 9 auszuführen. Somit ist die Fluidleitung 7 schließbar, indem die Fluidleitblende 18, nachdem die Verschlussaufnahme 2 mit dem Verschluss 3 zur Seite geschwenkt wurde, von der Seite zu einer Position über dem Nährmediumsträger 11 verschwenkt wird.

Weiterhin ist gezeigt, dass, im Gegensatz zu dem vorgehenden Ausführungsbeispiel, die Möglichkeit besteht, dass der Verschluss 3 in der kontrollierten Umgebung 5 außerhalb des Strömungswegs des Fluidstroms 17 positionierbar ist.

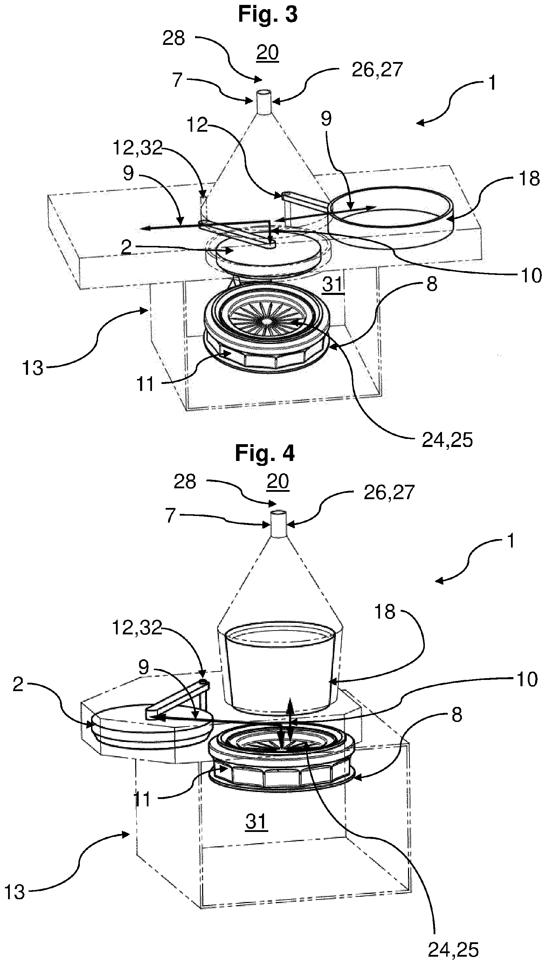

Fig. 3 zeigt eine dreidimensionale Darstellung einer alternativen Ausführung einer Vorrichtung 1 nach Fig. 2. Im Unterschied zu Fig. 2 ist hierbei zusätzlich ein Entnahmeraum 31 ausgebildet, der von dem Gehäuse 13 gebildet wird.

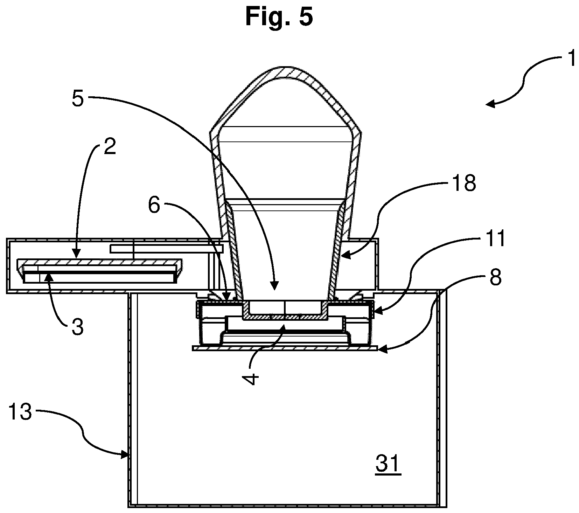

Fig. 4 und Fig. 5 zeigen eine weitere Ausführungsform gemäß den vorgehenden Ausführungsbeispielen, mit dem Unterschied, dass das Gehäuse 13 nicht zwei, sondern lediglich einen Platzhalter für die Verschlussaufnahme 2, die den Verschluss 3 umgreift, bereitstellt. Die Fluidleitblende 18 ist in diesem Ausführungsbeispiel nicht schwenkbar, sondern vertikal beweglich, um die Anbindung des Fluidstroms 17 zu bewirken.

Fig. 5 zeigt die Fluidleitblende 18 im angedockten Zustand.

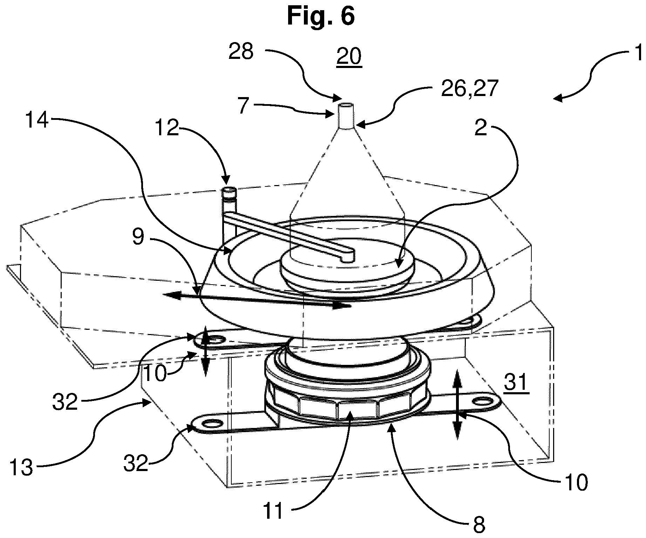

Fig. 6 zeigt im Unterschied zu den vorgehenden Ausführungsbeispielen eine elastische Membrane 14, die außerhalb eines Strömungswegs des Fluidstroms 17 zwischen einem Entnahmeraum 31 und der kontrollierten Umgebung 5 ausgebildet ist und einen Teilabschnitt der Einhausung 13 bildet. Über den Entnahmeraum 31 kann der Impaktor 29 entnommen und zugeführt werden.

Hierbei ist vorgesehen, dass die Membrane 4 zur Entfernung des Verschlusses 3 und/oder zur Ankopplung der Fluidleitung 7 von der Hebevorrichtung 32, die eine Hubbewegung 10 der Aufnahme 8 realisiert, mit dem Nährmedium 4, insbesondere mit dem Nährmediumsträger 11 verbindbar oder verbunden ist und vertikal bewegt wird.

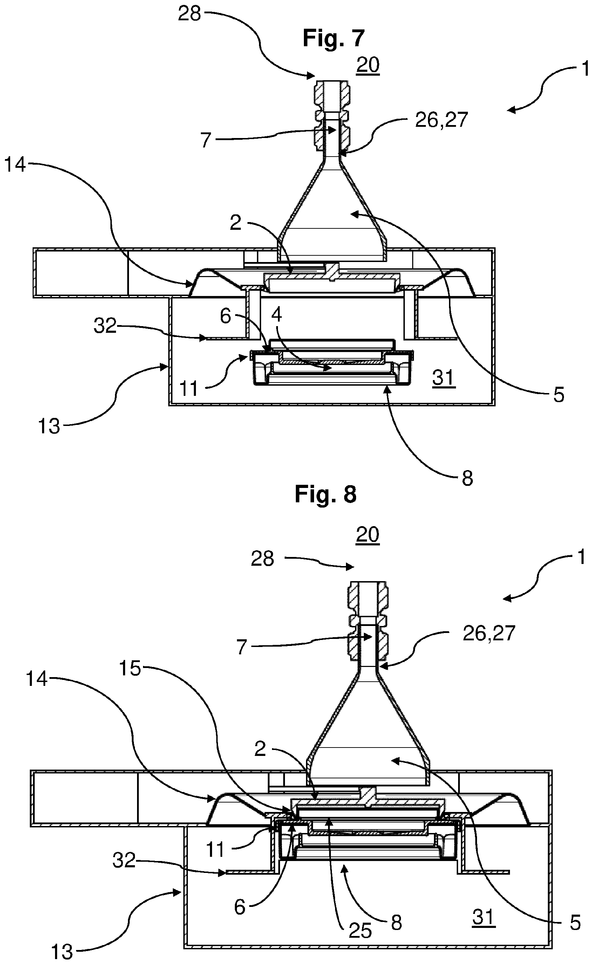

In Fig. 7 bis Fig. 10 sind dabei die durch die vertikale Bewegung der Aufnahme 8 mit der Membrane 14 möglichen Stellungen gezeigt.

In Fig. 7 ist dabei gezeigt, dass das Nährmedium 4, welches in dem Nährmediumsträger 11 positioniert ist, nicht an die Membrane 14 fluidisch angeschlossen ist. In dieser Stellung kann das Nährmedium 4 in die kontrollierbare Umgebung 5 eingebracht werden.

Anschließend kann, gemäß Fig. 8, das Nährmedium mit dem Verschluss 3, insbesondere der Nährmediumsträger 11, über die Hebevorrichtung 32 an die Verschlussaufnahme 2 angehoben werden.

Somit kann gesagt werden, dass eine fluidische Anbindung 27, die ein Leitungsstück 26 umfasst, mittels einer Kupplung 15, die eine Relativbewegung zwischen der Düsenplatte 25 und dem Leitungsstück 26 ausführt, hergestellt wird.

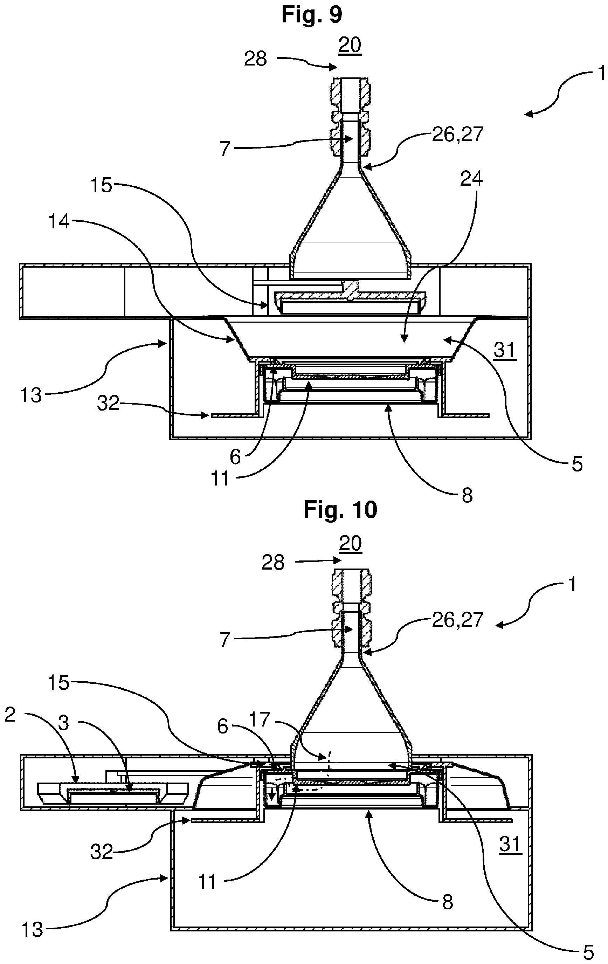

Fig. 9 zeigt, dass der Nährmediumsträger 11 anschließend wieder abgesenkt wird, wodurch über die Verbindung des Nährmediumsträgers 11 mit der elastischen Membrane 14 die elastische Membrane 14 ebenfalls abgesenkt wird.

Letztlich kann, gemäß Fig. 10, die Verschlussaufnahme 2, die den Verschluss 3 des Nährmediums 4 umfasst, über eine Schwenkbewegung 9 außerhalb des Strömungswegs des Fluidstroms 17 innerhalb der kontrollierten Umgebung 5 platziert und das Nährmedium 4 ohne Verschluss 3 präsentiert werden.

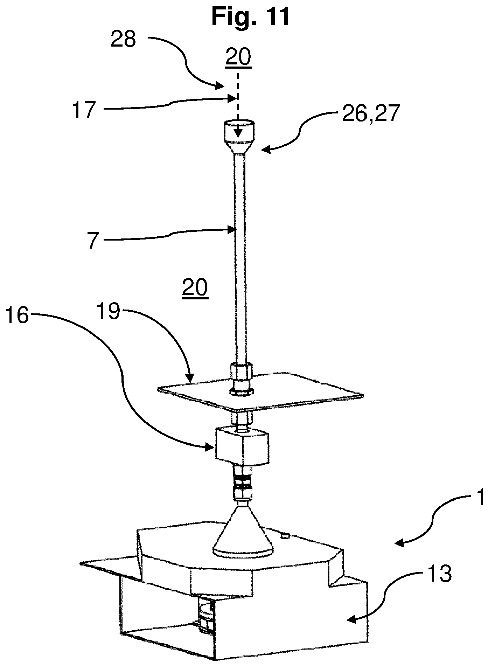

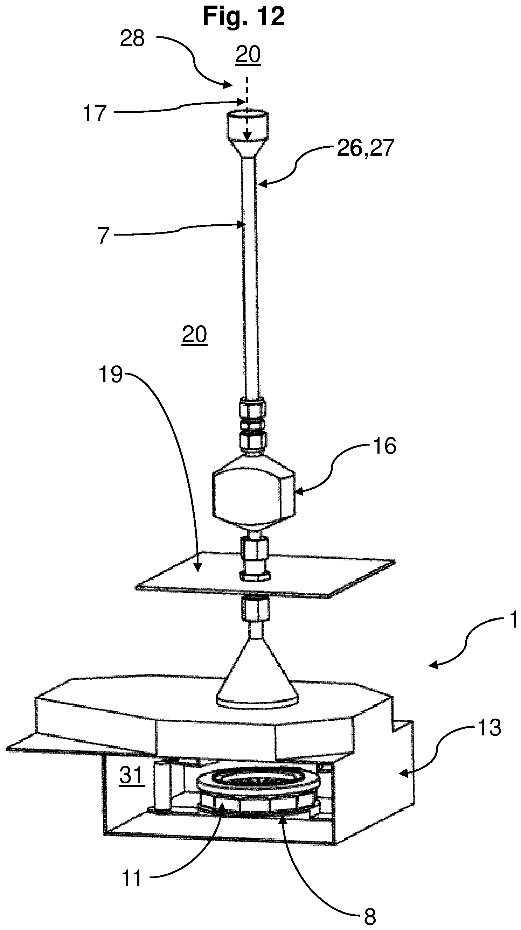

Fig. 11 und 12 zeigen die gesamte Vorrichtung 1, wobei die Fluidleitung 7 teilweise innerhalb des Reinraums 20 verläuft. Zudem ist ein Partikelzähler 16 gezeigt, der in Fig. 11 vor einer Bodenplatte 19 des Reinraums 20, zum Beispiel eines Isolators, und in Fig. 12 innerhalb des Reinraums 20 an der Fluidleitung 7 positioniert ist. Somit kann der aus dem Reinraum 20 über die Fluidleitung 7 abgeführte Fluidstrom 17 vor der Kontaktierung mit dem Nährmedium 4 durch den Partikelzähler 16 durchgeführt werden.

Bei weiteren Ausführungsbeispielen kann statt der Bodenplatte 19 auch eine Seitenwand und/oder eine Decke zur Durchführung der Fluidleitung 7 genutzt sein.

Eine weitere in diesem Ausführungsbeispiel nicht gezeigte Variante besteht zudem darin, den Fluidstrom 17 beispielsweise außerhalb des Reinraums 20 abzuzweigen, wobei der abgezweigte Fluidstrom 17 ebenfalls durch den Partikelzähler 16 geführt wird.

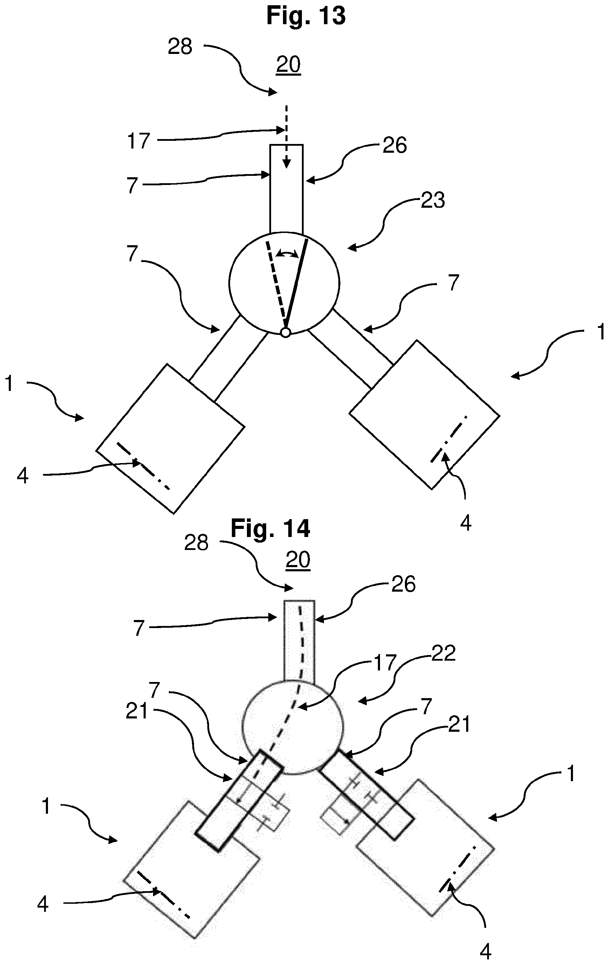

Die Fig. 13 und Fig. 14 zeigen einen prinzipiellen Aufbau zur Überwachung auf mikrobiologische Verunreinigungen in einem Reinraum 20, wobei, im Unterschied zu den vorhergehenden Ausführungsbeispielen, zwei separate Vorrichtungen 1 verwendet werden, die jeweils mit einer Fluidleitung 7 ausgebildet sind.

Der Fluidstrom 17, der aus dem Reinraum 20 zur Überwachung auf mikrobiologische Verunreinigungen abgeführt wird, kann somit in zwei verschiedene Vorrichtungen 1 entweder über eine stellbare Weiche 23 oder über eine Verzweigung 22 der Fluidleitung 7 geleitet werden.

Eine der beiden Vorrichtungen 1 präsentiert dabei ein Nährmedium 4, das der Fluidstrom 17, der aus dem Reinraum 20 abgeführt wird, kontaktiert. Sobald das Nährmedium 4, welches in einer der Vorrichtungen 1 präsentiert wird, gewechselt werden soll, kann der Fluidstrom 17, beispielsweise über die Weiche 23 oder über die Verzweigung 22 der Fluidleitung 7, welche vorzugsweise mit einem Ventil 21 ausgebildet ist, in die anderen Vorrichtung 1 umgeleitet werden, in die, vorzugsweise vor dem Umleiten des Fluidstroms 17, ein neues Nährmedium 4 eingelegt wird. Die Verzweigung 22 der Fluidleitung 7, die mit der Vorrichtung 1 verbunden ist, in welcher das Nährmedium 4 gewechselt werden soll, wird beispielsweise über das Ventil 21, welches sich dabei insbesondere in einer Sperrstellung befindet, abgesperrt. Das Ventil 21 befindet sich dabei zur Vermeidung von Toträumen möglichst nahe an der Verzweigung 22.Es kann somit gesagt werden, dass hierüber eine kontinuierliche Überwachung auf mikrobiologische Verunreinigungen realisierbar ist.

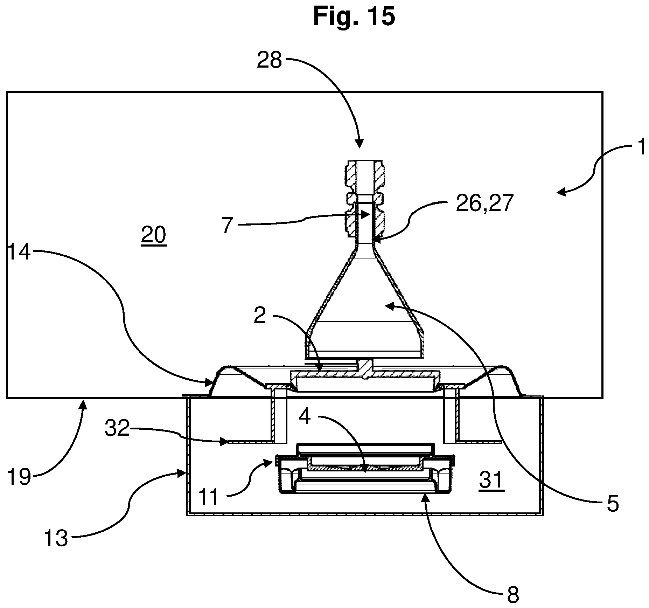

Fig. 15 zeigt die modifizierte Vorrichtung 1 gemäß Fig. 6 in einem eingebauten Zustand in einem Reinraum 20. Es zeigt sich, dass die kontrollierte Umgebung 5 von dem Reinraum 20 gebildet ist und dass die Membrane 14 als Teilabschnitt des Reinraums 20 ausgebildet ist. Zusätzlich ist zu erkennen, dass die kontrollierte Umgebung 5, bis auf die Fluidleitung 7, von dem Reinraum 20 abtrennbar ist.

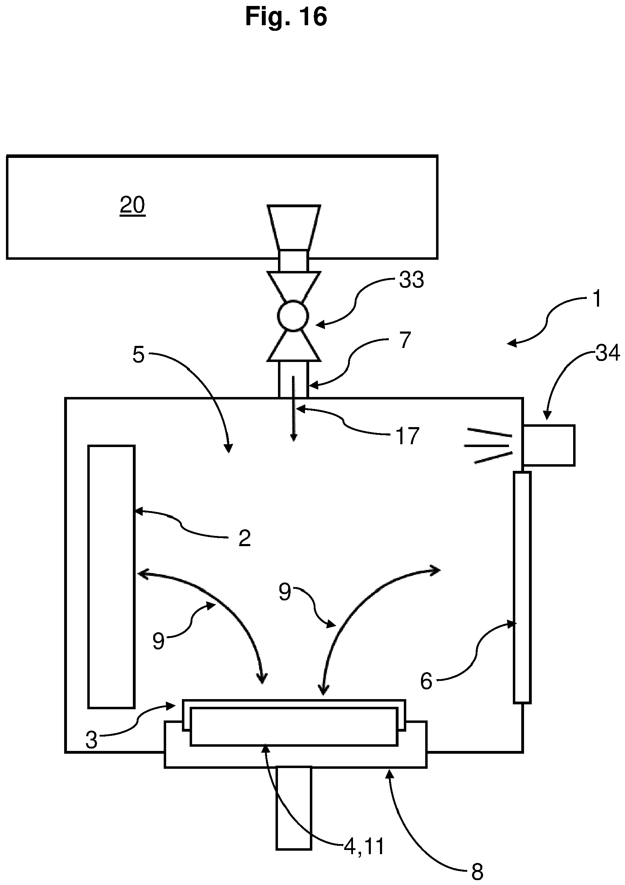

Fig. 16 zeigt eine weitere erfindungsgemäße Vorrichtung 1 mit einer seitlich die kontrollierte Umgebung 5 begrenzende, über eine Schwenkbewegung 9 klappbaren Schnittstelle 6, die zumindest innenseitig dekontaminiert wird, bevor die Schnittstelle 6 für den zu überwachenden Fluidstrom 17 zugänglich gemacht wird. Um einem Rückstrom des aus dem Reinraum 20, vorzugsweise Isolator, abgeführten Fluidstroms 17 entgegenzuwirken, ist an der Fluidleitung 7 eine Rückstromverhinderung, welche in diesem Ausführungsbeispiel als Kugelventil 33 zwischen der kontrollierten Umgebung 5 und dem Reinraum 20 ausgebildet ist, angebracht. In einem nicht gezeigten Ausführungsbeispiel ist anstelle des Kugelventils 33 eine andere Rückstromverhinderung, beispielsweise ein Membranventil oder eine andere, dem Fachmann bekannte, Absperrvorrichtung angebracht.

In diesem Ausführungsbeispiel wird das zur Dekontamination benötigte Dekontaminationsmittel der Schnittstelle 6 und der kontrollierten Umgebung 5 über eine Düse 34 zugeführt.

Zudem zeigt sich, ähnlich zu den vorhergehenden Ausführungsbeispielen, dass der Verschluss 3 über eine Schwenkbewegung 9 der Verschlussaufnahme 2 von dem Nährmediumsträger 11, der das Nährmedium 4 präsentiert, abgenommen oder auf dem Nährmediumsträger 11 zum Verschließen des Nährmediums 4 aufgesetzt wird.

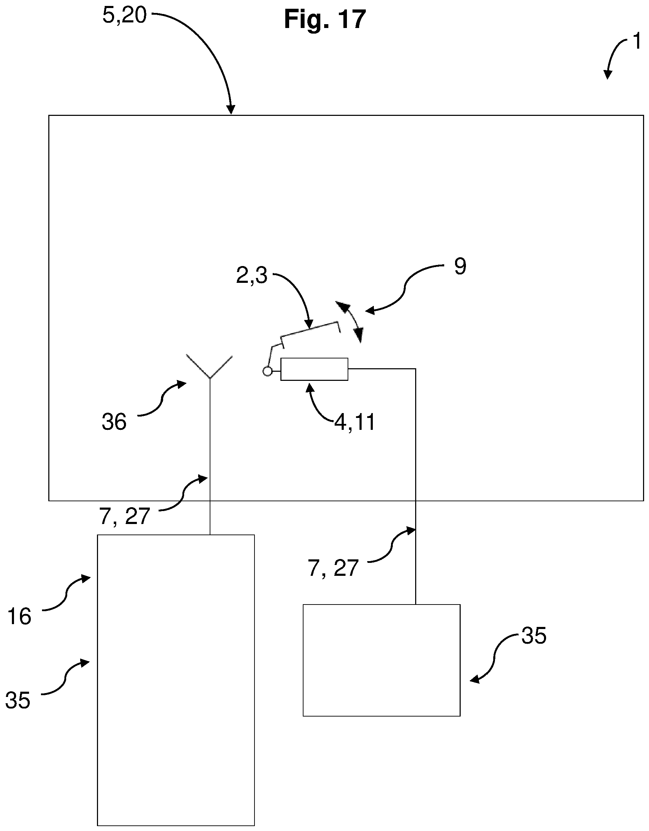

Figur 17 zeigt eine weitere Vorrichtung zur Überwachung auf mikrobiologische Verunreinigungen 1. Es zeigt eine Fluidleitung 7 bzw. fluidische Anbindung 27 mit einem Partikelzähler 16, der innerhalb der kontrollierten Umgebung 5, die in diesem Ausführungsbeispiel von dem Reinraum 20 selbst gebildet wird, Fluidproben, hier Gasproben, an einer Entnahmestelle 36 entnimmt. Hierbei wird ein kontrollierter Fluidstrom 17 über eine außerhalb des Reinraums 20 angeordnete (im Bild linken) Pumpe 35 angesaugt und somit dem Partikelzähler 16 zugeführt.

Zusätzlich befindet sich der Nährmediumsträger 11 mit dem darin präsentierten Nährmedium 4 ebenfalls innerhalb der kontrollierten Umgebung 5 bzw. Reinraum 20 parallel zum Partikelzähler 16. Der Nährmediumsträger 11 ist dabei mit einer weiteren außerhalb des Reinraum 20 angeordneten Pumpe 35 über eine weitere Fluidleitung 7 bzw. fluidische Anbindung 27 verbunden, über die der Fluidstrom 17 angesaugt wird, wodurch wiederum eine definierte Kontaktierung des Fluidstroms 17 des Reinraums 20 mit dem Nährmedium 4 möglich ist. Hierbei ist vorgesehen, dass zunächst eine kontinuierliche Überwachung auf mikrobiologische Verunreinigungen über den Partikelzähler 16 erfolgt, wobei das Nährmedium 4 von dem Verschluss 3 zunächst verschlossen ist und somit nicht präsentiert wird. Von der (im rechten Bild) Pumpe 35, die über die Fluidleitung 7 mit dem Nährmediumsträger 11 verbunden ist, wird somit zunächst kein Fluidstrom 17 angesaugt.

Sobald von dem Partikelzähler 16 Objekte detektiert wurden, wird der Verschluss 3 des Nährmediums 4 automatisch von der Verschlussaufnahme 2 über eine Schwenkbewegung 9 entfernt und das Nährmedium 4 präsentiert. Über die (rechte) Pumpe 35, die über die Fluidleitung 7 mit dem Nährmediumsträger 11 verbunden ist, wird somit der Fluidstrom 17 angesaugt, der somit wiederum das präsentierte Nährmedium 4 kontaktiert.

Es kann somit gesagt werden, dass das Nährmedium 4 erst dann in dem Fluidstrom 17 präsentiert wird, wenn von dem Partikelzähler 16 Verunreinigungen detektiert wurden. Das Nährmedium 4 kann nach der Präsentation in an sich bekannter Weise darauf untersucht werden, ob die Verunreinigung vermehrungsfähig ist. In einem nicht gezeigten Ausführungsbeispiel kann weiterhin vorgesehen sein, dass sich das Nährmedium 4 außerhalb des Reinraums 20 innerhalb einer kontrollierten Umgebung 5 befindet. Die Fluidleitung 7 bzw. Fluidische Anbindung 27 verbindet dabei, gemäß Fig. 16, den Reinraum 20 mit einer kontrollierten Umgebung 5, beispielsweise wie bei den vorangehenden Ausführungsbeispielen, und ist zusätzlich mit einem schaltbaren Ventil 21 ausgebildet. Sobald der Partikelzähler 16 mikrobiologische Verunreinigungen detektiert hat, wird das Ventil 21 in eine Offenstellung geschaltet, wodurch das Nährmedium 4 wiederum automatisch präsentiert und von dem Fluidstrom 17 kontaktiert wird.

Das Nährmedium 4 und der Partikelzähler 16 sind bei einem weiteren Ausführungsbeispiel an eine gemeinsame Pumpe 35 angeschlossen. Das Nährmedium 4 kann somit in einem schaltbaren Bypass zum Fluidstrom des Partikelzählers 16 angeordnet sein.

Das Nährmedium 4 kann bei einem weiteren Ausführungsbeispiel auch mit einer vorgeschalteten, insbesondere abkoppelbaren Fluidleitung 7 (vgl. bspw. Fig. 12) versehen sein.

Erfindungsgemäß wird somit zur Überwachung auf mikrobiologische Verunreinigungen vorgeschlagen, dass ein Fluidstrom 17 über eine Fluidleitung 7 aus einem Reinraum 20 in eine kontrollierte Umgebung 5 abgeführt wird, wobei in der kontrollierten Umgebung 5 ein Nährmedium 4 präsentiert und mit dem der Fluidstrom 17 kontaktiert wird und anschließend so aus der kontrollierten Umgebung 5 entnommen wird, dass ein Reinraumprozess nicht unterbrochen werden muss.

### Bezugszeichenliste

- 1: Vorrichtung zur Überwachung auf mikrobiologische Verunreinigungen
- 2: Verschlussaufnahme
- 3: Verschluss
- 4: Nährmedium
- 5: kontrollierte Umgebung
- 6: Schnittstelle
- 7: Fluidleitung
- 8: Aufnahme
- 9: Schwenkbewegung
- 10: Hubbewegung
- 11: Nährmediumsträger
- 12: Schwenkvorrichtung
- 13: Einhausung
- 14: Elastische Membrane
- 15: Kupplung
- 16: Partikelzähler
- 17: Fluidstrom
- 18: Fluidleitblende
- 19: Bodenplatte Reinraum
- 20: Reinraum
- 21: Ventil
- 22: Verzweigung
- 23: Weiche
- 24: Öffnung
- 25: Düsenplatte
- 26: Leitungsstück
- 27: Fluidische Anbindung
- 28: Entnahmeort
- 29: Impaktor
- 30: Sauganschluss
- 31: Entnahmeraum
- 32: Hebevorrichtung
- 33: Kugelventil
- 34: Düse
- 35: Pumpe
- 36: Entnahmestelle

## Patentansprüche

1. Verfahren zur Überwachung auf mikrobiologische Verunreinigungen in einem Reinraum (20), vorzugsweise Isolator, wobei ein Fluidstrom (17), insbesondere eine Luftströmung, aus dem Reinraum (20) abgeführt wird, **dadurch gekennzeichnet, dass** der Fluidstrom (17) ein Nährmedium (4) in einer kontrollierten, außerhalb des Reinraums (20) angeordneten, vorzugsweise in einer durch eine Fluidleitung (7) verbundenen Umgebung (5) und/oder von dem Reinraum (20) abtrennbaren Umgebung (5) kontaktiert, wobei das Nährmedium (4) zur Überwachung auf mikrobiologische Verunreinigungen verwendet wird.

2. Verfahren nach Anspruch 1, wobei eine die kontrollierte Umgebung (5) begrenzende Schnittstelle (6) zumindest innenseitig dekontaminiert wird, insbesondere bevor die Schnittstelle (6) für den zu überwachenden Fluidstrom (17) zugänglich gemacht wird und/oder wobei an der Fluidleitung (7) eine Verzweigung (22) oder eine Weiche (23) ausgebildet ist, insbesondere wobei der Fluidstrom (17) steuerbar ist.

3. Verfahren nach einem der voranstehenden Ansprüche, wobei zur Dekontamination der Schnittstelle (6) und/oder der kontrollierten Umgebung (5) ein Dekontaminationsmittel, insbesondere ein Gas bzw. Aerosol, vorzugsweise Wasserstoffperoxid, zugeführt wird, insbesondere wobei das Gas bzw. Aerosol aus dem Reinraum (20) und/oder aus einer separaten Dekontaminationsquelle zugeführt wird.

4. Verfahren nach einem der voranstehenden Ansprüche, wobei der Fluidstrom (17) an einer Prozessstelle innerhalb des Reinraums (20), an der vorzugsweise ein Reinraumprozess durchgeführt wird, aus dem Reinraum (20) abgeführt wird, und/oder wobei der Fluidstrom (17) in einer Fluidleitung (7), welche zumindest teilweise innerhalb des Reinraums (20) verläuft, dem Nährmedium (4) zugeführt wird.

5. Verfahren nach einem der voranstehenden Ansprüche, wobei der Fluidstrom (17) durch einen Partikelzähler (16) zur Erfassung einer Partikelanzahl und/oder Partikelgröße geführt wird, insbesondere wobei anschließend derselbe Fluidstrom (17) zum Nährmedium (4) geführt wird, und/oder wobei ein Teilvolumen des Fluidstroms (17) abgezweigt wird, insbesondere wobei der abgezweigte Fluidstrom (17) an einen Partikelzähler (16) zur Erfassung einer Partikelanzahl überführt wird und/oder wobei die Abzweigung außerhalb des Reinraums (20) stattfindet.

6. Verfahren nach einem der voranstehenden Ansprüche, wobei das Nährmedium (4) von außen in die kontrollierte Umgebung (5) eingebracht und an einer Aufnahme (8) positioniert wird, insbesondere wobei nach dem Einbringen die kontrollierte Umgebung (5) dekontaminiert wird und/oder wobei vor einem Austausch des Nährmediums (4) eine Rückströmverhinderung, insbesondere ein Ventil 21, vorzugsweise zwischen der kontrollierten Umgebung (5) und dem Reinraum (20) aktiviert wird und/oder wobei nach dem Einbringen ein Verschluss (3) des Nährmediums (4) entfernt und in der kontrollierten Umgebung (5) aufbewahrt wird, insbesondere wobei der Verschluss (3) außerhalb eines Strömungswegs des Fluidstroms (17) aufbewahrt wird.

7. Verfahren zur Überwachung auf mikrobiologische Verunreinigungen in einem Reinraum (20), wobei ein Fluidstrom (17), insbesondere eine Luftströmung, aus dem Reinraum (20) abgeführt wird, wobei der Fluidstrom (17) ein Nährmedium (4) kontaktiert, wobei das Nährmedium (4) zur Überwachung auf mikrobiologische Verunreinigungen verwendet wird, insbesondere nach einem der vorangehenden Ansprüche, wobei eine Fluidleitung (7), die den Fluidstrom (17) zum Nährmedium (4) bringt, an das Nährmedium (4) angeschlossen wird, nachdem ein Verschluss (3) des Nährmediums entfernt wurde.

8. Verfahren nach einem der voranstehenden Ansprüche, wobei der Verschluss (3) über eine Transferbewegung, insbesondere eine vorzugsweise horizontale Schwenkbewegung (9) und/oder eine Hubbewegung (10), entfernt wird und/oder wobei das Nährmedium (4) nach erfolgter Kontaktierung mit dem Fluidstrom (17) aus der kontrollierten Umgebung (5) entfernt wird, insbesondere durch eine Bewegung der Aufnahme (8).

9. Verfahren nach einem der voranstehenden Ansprüche, wobei das Nährmedium (4), insbesondere auf einem Nährmediumsträger (11), zur Entfernung und/oder zur Anbringung des Verschlusses (3) und/oder zur Ankopplung der Fluidleitung (7) von einer Hebevorrichtung (32), insbesondere die mit einer Membrane (14) verbindbar oder verbunden ist, vorzugsweise vertikal bewegt wird.

10. Verfahren nach dem Oberbegriff von Anspruch 1 oder nach einem der voranstehenden Ansprüche, **dadurch gekennzeichnet, dass** in dem Fluidstrom (17) oder einem weiteren Fluidstrom (17) eine Partikeldetektion durchgeführt wird und ein oder das Nährmedium (4) erst und/oder automatisch in dem Fluidstrom (17) präsentiert wird, wenn die Partikeldetektion ein vorzugsweise vermehrungsfähiges und/oder lebendes Objekt detektiert hat, insbesondere durch Umleiten des Fluidstroms (17), Öffnen einer den Fluidstrom (17) leitenden Fluidleitung (7) und/oder Starten des Fluidstroms (17).

11. Vorrichtung (1) zur Überwachung auf mikrobiologische Verunreinigungen in einem Reinraum (20), insbesondere zur Durchführung eines wie zuvor beanspruchten Verfahrens, aufweisend eine Einhausung (13), die die oder eine kontrollierte Umgebung (5) umschließt, die oder eine Aufnahme (8) zum Präsentieren eines oder des Nährmediums (4) in der kontrollierten Umgebung (5), eine fluidische Anbindung (27) zur Verbindung mit dem Reinraum (20) und eine Schnittstelle (6), welche die kontrollierte Umgebung (5) begrenzt, insbesondere zur Bereitstellung des Nährmediums (4).

12. Vorrichtung (1) nach dem vorangehenden Anspruch, wobei die fluidische Anbindung (27) eine Verzweigung (22) oder eine Weiche (23) hat, die dazu ausgebildet ist, einen Fluidstrom (17) umzulenken und/oder wobei eine Dekontaminationsvorrichtung ausgebildet ist, durch welche zumindest die Schnittstelle (6), insbesondere die kontrollierte Umgebung (5), dekontaminierbar ist und/oder wobei die Schnittstelle (6) als Teil der Einhausung (13), insbesondere als Teil einer Verschlussvorrichtung der Einhausung (13) ausgebildet ist.

13. Vorrichtung (1) nach einem der voranstehenden, auf eine Vorrichtung (1) gerichteten Ansprüche, wobei die kontrollierte Umgebung (5) eine Zuleitung und/oder eine Ableitung für ein Dekontaminationsmittel der Dekontaminationsvorrichtung hat, und/oder wobei die Dekontaminationsvorrichtung als Teil der Vorrichtung (1) ausgebildet ist.

14. Vorrichtung (1) nach dem voranstehenden Anspruch, wobei die Aufnahme (8) in der kontrollierten Umgebung (5) ausgebildet ist, insbesondere wobei die Aufnahme (8) dazu eingerichtet ist, die Vorrichtung (1) nach außen zu öffnen und/oder wobei die Vorrichtung (1) eine Hebevorrichtung (32) aufweist.

15. Vorrichtung (1) zur Überwachung mikrobiellen Wachstums in einem Reinraum (20), insbesondere zur Durchführung eines wie zuvor beanspruchten Verfahrens und/oder nach einem der auf eine Vorrichtung (1) gerichteten vorangehenden Ansprüche, umfassend die oder eine Aufnahme (8) zum Präsentieren eines oder des Nährmediums (4), eine fluidische Anbindung (27), insbesondere von einem Entnahmeort (28) im Reinraum (20), die an das Nährmedium (4) ankoppelbar ist und ein Mittel zum vorzugsweise ferngesteuerten Entfernen eines Verschlusses (3) des Nährmediums (4) bevor die fluidische Anbindung (27) angekoppelt ist.

16. Vorrichtung (1) nach einem der voranstehenden, auf eine Vorrichtung (1) gerichteten Ansprüche, wobei eine elastische Membrane (14) außerhalb eines Strömungswegs des Fluidstroms (17) zwischen dem Isolator und der kontrollierten Umgebung (5) ausgebildet ist und/oder wobei die Membrane (14) als Teilabschnitt der oder einer Einhausung (13) der oder einer kontrollierten Umgebung (5) oder des Reinraums (20) ausgebildet ist und/oder wobei wobei die Vorrichtung (1) eine Transfervorrichtung, insbesondere Schwenkvorrichtung (12), aufweist, die mit einer Verschlussaufnahme (2) ausgebildet ist.

17. Vorrichtung (1) nach einem der voranstehenden, auf eine Vorrichtung (1) gerichteten Ansprüche, wobei die Vorrichtung (1) die oder eine Fluidleitung (7) umfasst, wobei insbesondere die Schnittstelle (6) als Teil der Fluidleitung (7) ausgebildet ist, und/oder wobei ein Strömungsweg des Fluidstroms (17) durch die Fluidleitung (7) zwischen einer der Vorrichtung (1) abgewandten Öffnung (24) der Fluidleitung (7) und der Aufnahme (8) weniger als ein Meter, vorzugsweise weniger als zwei oder drei Meter, insbesondere weniger als vier Meter beträgt.

18. Vorrichtung (1) nach einem der voranstehenden Ansprüche, wobei die Fluidleitung (7) schließbar ist, insbesondere, wenn die Transfervorrichtung in einer entfernten Position angeordnet ist und/oder wobei im Strömungsweg vorgeschaltet dem Nährmedium (4) der oder ein Partikelzähler (16) angeordnet ist und/oder dass der Fluidstrom (17) abgezweigt wird.

19. Vorrichtung (1) nach einem der voranstehenden, auf eine Vorrichtung (1) gerichteten Ansprüche, wobei die fluidische Anbindung (27) mit einer Einrichtung zur Rückströmverhinderung, insbesondere einem Ventil (21), ausgebildet ist und/oder wobei die Vorrichtung (1) eine Pumpe (35), insbesondere zum Einbringen des Fluidstroms (17) in die kontrollierte Umgebung (5), aufweist.

20. Vorrichtung (1) nach einem der voranstehenden, auf eine Vorrichtung (1) gerichteten Ansprüche, wobei die Vorrichtung (1) wenigstens einen Sensor zur Erfassung der oder einer Kenngröße des Fluidstroms (17) hat, und/oder wobei die Vorrichtung (1) eine Steuervorrichtung zur Regulierung des Fluidstroms (17) hat.

21. Verwendung, insbesondere innerhalb eines Reinraums (20) und/oder innerhalb einer kontrollierten Umgebung (5), eines verpackten Nährmediums (4), insbesondere Impaktors (29), zur Präsentation des Nährmediums (4) in einem Fluidstrom (17), wobei der Fluidstrom (17) von einem Entnahmeort (28) aus einem Reinraum (20), insbesondere Isolator, entnommen wurde, insbesondere wobei der Impaktor (29) eine Düsenplatte (25) hat, hinter der das Nährmedium (4) angeordnet ist, wobei das Nährmedium (4), insbesondere hinter der Düsenplatte (25), vor Gebrauch durch einen Verschluss (3), insbesondere einen Deckel, abgedeckt ist, **dadurch gekennzeichnet, dass** der Verschluss (3) mit einer Verschlussaufnahme (2) gegriffen und von dem Nährmedium (4), insbesondere der Düsenplatte (25), entfernt wird und dass eine fluidische Anbindung (27) von dem Entnahmeort (28) zu dem Nährmedium (4), insbesondere der Düsenplatte (25), hergestellt wird.

22. Verwendung eines Impaktors (29) nach einem der vorangehenden, auf eine Verwendung eines Impaktor (29) gerichteten Anspruchs, **dadurch gekennzeichnet, dass** der Reinraum (20) und/oder die kontrollierte Umgebung (5) eine mit der Verschlussaufnahme (2), welche eine Außenseite des Verschlusses (3) abdeckt, verschließbare Öffnung (24) hat.

23. Verwendung eines Impaktors (29) nach einem der vorangehenden, auf eine Verwendung eines Impaktor (29) gerichteten Ansprüche, **dadurch gekennzeichnet, dass** der Impaktor (29) nach einem Exponieren des Nährmediums (4) von dem Fluidstrom (17) entfernt und einer Inkubation zugeführt wird, insbesondere wobei vor dem Entfernen das Nährmedium (4) verschlossen wird und/oder dass die Verschlussaufnahme (2) vor einer Kontaktierung des Verschlusses (3) einen Abschluss einer kontrollierten Umgebung (5) bildet, insbesondere auf einer von dem Verschluss(3) abgewandten Seite.

24. Verwendung eines Impaktors (29) nach einem der vorgehenden Ansprüche, **dadurch gekennzeichnet, dass** die fluidische Anbindung (27) ein Leitungsstück (26) umfasst und/oder dass die fluidische Anbindung (27) mittels einer Kupplung (15) hergestellt wird, die eine Relativbewegung zwischen der Düsenplatte (25) und einem Leitungsstück (26) ausführt.
